# EUROPEAN PATENT APPLICATION

(11) **EP 2 145 896 A2**
(43) Date of publication of application: **20.01.2010**
(21) Application number: 09175278.2
(22) Date of filing: 12.11.2003
(51) Int. Cl.: C07K 14/415, C07K 1/14, A61K 38/16

(54) **Recovery of a heat shock protein**

(62) Divisional of application: 03078579.4
(71) Applicant: Alfa Biogene International B.V., 48455 Bad Bentheim (DE)
(72) Inventor: Bukau, Bernd, 69120, Heidelberg (DE); Mayer, Mattias P., 69120, Heidelberg (DE)
(74) Representative: Hatzmann, Martin

(57) **Abstract**

The present invention relates to a plant heat shock protein showing chaperone activity in combination with a vertebrae cochaperone for use as a medicament.

## Description

The present invention relates to a method for recovering a heat shock protein (HSP), in particular an HSP from a plant. The invention further relates to an HSP from a plant.

Stress proteins (also called chaperone proteins) are formed by micro-organisms, plants and animals especially when, as a result of a change in the environment such as exposure to heat, radiation or chemicals, so-called stress-susceptible genes are expressed. According to current insights, such proteins can contribute to a protection against detrimental effects resulting from such environmental changes. For that reason, the use of stress proteins is in the centre of interest of, *inter alia,* medicine, molecular biology, the cosmetic industry and the industry producing plant protection products.

It is known that stress proteins have a number of important natural therapeutic functions in plants and animals, people included. These proteins are involved as regulator in protein synthesis and protein folding. In addition, an important function lies in the regulation of gene function during growth, but also during cell death. The stress proteins are capable of stimulating both human and animal immune systems, and clinical research has shown that the chaperone proteins contribute positively in the fight against cancer.

However, the availability of stress proteins such as HSP70 is a problem as they are often isolated from bovine brain. In addition to the slight amounts that can be isolated there from and the accompanying extreme high cost price, the possible presence of BSE in brain is a large impediment to the use of these proteins. HSP70 can also be produced from micro-organisms and cancer cells, but these production methods too are expensive and only give a low yield.

Alternatives to the production of the stress proteins, in particular for medical uses, lie in the field of the production of fusion proteins with the aid of expression systems. Possible problems that may continue to exist are the high cost price and the small amounts that can be produced.

Stress proteins of vegetable origin could offer an alternative. As the production of stress proteins depends on the condition the cell is in, a change in the circumstances outside the cell can tremendously increase the synthesis of the stress proteins in the plant cell.

In WO 00/70931, a method is described for the preparation of stress proteins from vegetable material. By giving the plants a heat treatment, the amount of stress protein can be increased. This publication further describes a method wherein the sap of the plant is heated to 95°C. However, it has been found that stress proteins such as HSP70 already denaturate in a temperature treatment of only 40 - 60°C (see Example 3), so that such a method is not desirable for the isolation of such stress proteins.

A disadvantage of the use of plants as a source of stress proteins is the large amount of other proteins (bulk proteins) and other bulk compounds present. As a result, the purification of stress proteins is difficult and also difficult to scale up, because the standard purification techniques such as ultrafiltration and column chromatography are hindered by the high concentrations of other proteins. Generally, the stress proteins / bulk proteins ratio is so low that a thorough separation of bulk proteins from the stress protein fraction is required to obtain a product with a suitable amount of stress protein without obtaining an excess of bulk proteins. When using an unpurified stress protein product in a food product or pharmaceutical use, the amount of product to be administered to obtain a desired amount of stress protein must be so high that an excess of bulk proteins and other bulk compounds such as polyphenols would be introduced into the food product or pharmaceutical preparation. Moreover, bulk proteins can have a negative effect on the activity of the stress protein, for instance because the stress protein binds to a bulk protein. Also, bulk proteins (such as proteolytic enzymes) can reduce the stability of a stress protein product.

WO 02/98910 describes a method to separate bulk proteins selectively from stress proteins by means of precipitation, which is realized by a pH-reduction. However, a drawback of reducing the pH, is the increased chance of release and activation of proteolytic enzymes, in particular when the stress proteins are isolated from a plant material, which may be detrimental to the efficiency of the process.

A problem often encountered with known methods to recover HSP's from plant include a low yield, a low purity and/or a low activity, after recovery.

In particular heat treatment and acid treatment have been found to be factors that can be detrimental to the activity of the HSP's.

Known methods for obtaining HSP with a high yield and purity, are generally not suitable for scaling up to an industrial scale.

Thus a need remains for alternative methods of recovering HSP's and for HSP's with an improved or different activity.

It has now been found that such an alternative method is provided by a specific multi step separation technique.

Accordingly, the present invention relates to a method for recovering a stress protein, in particular a heat shock protein, wherein a fluid comprising the stress protein, in particular a fluid from a plant or a plant extract, is subjected to a precipitation step -which may be a differential precipitation step - whereafter a HSP rich fraction (such as the last precipitate) is subjected to one or more further separation procedures (such as chromatography).

In particular, the present invention relates to a method for recovering a heat shock protein, comprising subjecting a fluid containing the heat shock protein to a) precipitation (such as a differential precipitation
wherein in general eventually a HSP containing material is precipitated), b) at least one ion exchange separation (after reconstituting the HSP containing material obtained in step a) and c) at least one affinity separation, which affinity separation is preferably a nucleotide-based separation.

Figure 1 shows a flow chart summarizing a suitable procedure for the purification of an HSP protein from plants.

Figure 2 shows an overview over the purification of Lucerne HSP70. The arrowheads indicate the position of Hsp70s. The numbers indicate the molecular weight of standard proteins.

Figure 3 shows the assessment of the activity of lucerne HSP70 and comparison with human HSP70. A, Steady-state.

A method according to the invention has been found suitable for convenient upscaling to an industrially attractive level. It has been found that in accordance with the invention a stress protein, in particular a HSP, can be obtained with a very high purity. Even for plant HSP's it has been found feasible to recover an HSP with more than 90 wt.% purity.

Further, a method according to the invention has been found to provide purified HSP in a high yield.

The combination of ion-exchange with affinity separation after precipitation has surprisingly been found an efficient way to remove bulk compounds from the HSP containing fluid to such an extent that they do not or not substantially adversely effect to recovery process (*e.g.* due to adherence of such compounds to the affinity material). In particular, it has been found that enzymes such as phosphatase, nucleotide binding proteins, nucleotide hydrolases and kinases may interfere with the affinity separation, if prior to the affinity separation, no ion exchange separation is carried out.

As referred to herein, bulk compounds are compounds that are contained in a large amount (compared to the HSP) in the liquid from which the desired stress protein can be produced, such as proteins and other peptidic compounds and other contaminants that are not stress proteins. Typical examples of bulk compounds are chlorophyll and proteins such as complexes of chlorophyll with "chlorophyll binding proteins", rubisco, enzymes (for instance proteases, phosphatases and polyphenol oxidases) and other proteins present in the liquid to such a degree that they impede efficient production of the stress protein. Inconvenient proteins, such as rubisco, "chlorophyll binding protein" and chlorophyll protein complexes are referred to herein as bulk proteins.

It has further surprisingly been found that a method according to the invention is suitable to isolate a heat shock protein (HSP) from a plant which HSP is capable to interact with a cochaperone of a vertebrate, in particular a mammal, more in particular a human. Preferably such a HSP has a molecular weight in the range of 20-130 kD. A method according to the invention has been found particularly suitable to recover a HSP60, HSP70 or HSP90.

It is surprising that a plant-HSP is capable of interacting with a vertebrate cochaperone as it is generally considered to be essential for a plant HSP to function in combination with a plant cochaperone in order to show activity such as ATPase activity and/or chaperone activity.

A plant-HSP showing chaperone activity with a cochaperone of a vertebrate has not been reported, thus far.

Accordingly, the present invention relates to a heat shock protein of a plant (a plant-HSP) showing chaperone activity in combination with a cochaperone of a vertebrate, in particular of a mammal, more in particular of a human, preferably under test conditions as defined below.

A preferred plant-HSP, in particular a preferred plant-HSP70, is found to have an amino acid sequence that corresponds for at least about 70 %, more preferably for at least about 80 %, even more preferably for about 90-100 % with the following amino acid sequence:
VX4TVPAYFNDX5QRQATK
wherein X4 is an amino acid residue, preferably V or I and X5 is an amino acid residue, preferably: S or A.

An amino acid sequence as shown above is considered to play a role in binding a cochaperone, preferably DnaJ. In particular an HSP wherein X4, X5 are respectively V, S; V, A; I, S or I, A is thought to act very satisfactory in cooperation with a cochaperone.

It is possible that a number, *e.g.* one, two or three, of the specified amino acid residues are replaced by another amino acid (two replacements corresponding to the amino acid being for about 90 % identical)

It is contemplated that a preferred plant-HSP according to the invention, in particular an HSP70, comprises the following amino acid sequence or is at least for about 70 %, preferably for at least 80 % more preferably for at least about 90 % identical to the following sequence:
GIDLGTTYSCVGV*W*X1X2DRVEIIX3NDQGNRTT
   wherein
X1 represents a natural amino acid residue, preferably Q, M or R.
X2 represents a natural amino acid residue, preferably H or N.
X3 represents a natural amino acid residue, preferably A or P.

Very good results have been achieved with an HSP, such as an HSP70, wherein X¹ is Q, X² is H and/or X³ is A.

An HSP with a sequence as defined above has thought to be very effective in ATP binding and hydrolysis. It is possible that a number, *e.g.* one, two or three, of the specified amino acid residues are replaced by another amino acid (three replacements corresponding to the amino acid being for about 90 % identical). In case a number of the amino acids is replace, it is preferred though that the bold and italicized amino acids on opposite sides of X¹ and X² (W respectively D and R, which are considered to be plant specific) are not replaced by any other amino acid.

A plant-HSP in accordance with the invention is very interesting for use as a medicament. In particular, it can perform a function of an HSP native to the patient which is treated with a medicament comprising a plant-HSP of the invention.

A very interesting feature of an HSP according to the invention is its capacity to stimulate refolding of denatured proteins, in the presence of a cochaperone, naturally present in the subject to be treated, or a recombinant cochaperone analogue of the naturally present cochaperone.

A plant-HSP according to the invention or an HSP obtained by a method according to the invention, may thus be used in *in vivo* protein repair.

Preferred medical indications which may be treated with a medicament comprising an HSP according to the invention include an indication selected from the group consisting of cancer, for instance cervical cancer and skin cancer, neurodegenerative diseases, such as Creutzfeld Jacob Disease, inflammatory and necrotic processes, autoimmune diseases,and of regenerative processes after injuries, lesions and hypoxic stress conditions..

In a medicament, the HSP may suitably be present in combination with a pharmaceutical acceptable carrier.

Preferably a medicament according to the invention comprises a cochaperone for the invention. Much preferred, in particular in case a HSP70 is present in the medicament, is a DnaJ cochaperone or a plant homologue thereof. Highly suitable examples of DnaJ are Hdj1 and Hdj2.

The amount of the cochaperone can be chosen within wide limits. Very suitable is a molar ratio of cochaperone to HSP, in particular HSP70, of at least about 0.1. The molar ratio preferably is at most about 1 for practical reasons.

The source for the HSP may be any. Preferably the source is a harvested plant material. Very suitable are plant materials from lucerne (alfalfa), a cereal (for instance barley), soy, a grass (for instance oat), beet, potato, clover or a water plant (for instance an alga). This technique can also be used for stress protein (such as HSP's) from yeasts, fungi and bacteria. Particularly good results are obtained with stress proteins from at least one plant selected from lucerne, clover, oat and barley.

Preferably, leaves of the plant are used as source for one or more stress proteins. Particularly suitable are beet tops, lucerne leaves, barley leaves, oat leaves and potato tops. In addition to the particular suitability of such leaf material as source for stress proteins, their use also offers the possibility to use such leaf material, which is normally a waste product, in a useful manner.

The plant can be grown in a conventional manner. Very good results have also been obtained with a plant grown in the dark in light-deficient conditions, or, conversely, in conditions rich in light. The choice of the light conditions can influence the formation of specific bulk proteins.

By growing the plant, for instance barley, in the dark or under conditions rich in light, it is possible to obtain plant material with a relatively low chlorophyll content, which yields a favourable base material for the preparation of a stress protein-containing liquid.

The plant material may have been treated after harvesting by a HSP inducing treatment. Examples of such treatments are known in the art, *e.g.* from WO 00/70931 or WO 02/98910.

A heat treatment may be carried out by subjecting harvested plant material to a temperature in the range of 20 to 60 °C, preferably 30 to 50 °C more preferably 35 to 45°C. The duration may be chosen in a wide range, *e.g.* from 0.1-100 hours, preferably 1-12 hours.

In a further aspect of the invention harvested plant material may be subjected to an atmosphere having an increased CO₂ concentration, in order to provide a plant material with an increased HSP concentration. This HSP may be recovered from the material by any suitable means or the plant material may be used as such.

Subjecting harvested plant material to a heat-treatment in an atmosphere with an increased CO₂-concentration, has been found to have a stimulating effect on the HSP production in the material, in particular when CO₂ concentration in the atmosphere is 1-100 vol %, preferably 5-100 vol. %, more preferably 10-100 vol %. The balance is preferably formed by one or more of the usual other components of air (such as oxygen, nitrogen, noble gasses).

In principle, any liquid with an HSP can be used as a source. Particularly suitable is for instance the sap of the plant obtained by pressing vegetable material or an extract of a vegetable material. Suitable preparation methods for such a liquid are known, for instance from WO 00/70931 and S. Lewis et al, Ecotoxicology 8, 351 - 368 (1999). Extraction can for instance take place with water or another solvent in which stress proteins dissolve. The solvent preferably comprises at least one additive selected from buffers ( preferably buffering the solvent at an alkaline pH) protease inhibitors such as phenylmethylsulfonylfluorid (PMSF), Pefabloc SC, leupeptide, pepstatin, protein stabilisers such as mono- and divalent metal ions (*e.g*. Na⁺, K⁺, Mg²⁺, Ca²⁺, Mn²⁺), antioxidants such as ascorbate, dithiothreitol, dithioerythritol, β-mercaptoethanol, sodium dithionite, sodium diethyldithiocarbamate *etc.* and anti-denaturants such as glycerol, mono-, di-, and oligosaccharides (*e.g*. glucose, sucrose, trehalose), sugar alcohols (*e.g.* sorbitol, inositol), ethylenglycol and polyethyleneglycol (in particular having a molecular weight in the range of 2 to 40 kDa), chelating agents such as EDTA and EGTA and the like.

The skilled person will know how to choose suitable concentrations and combinations of said additives, based upon common general knowledge and the information disclosed herein.

As a guidance, typical concentrations for protease inhibitors may be about 0.05 µg/ml to about 1 mg/ml, for divalent metal ions about 0.1 to about 10 mM, for antioxidants about 0.1 to about 30 mM, anti-denaturants about 5 to about 30 % (w/v), and for the chelating agents about 0.1 to about 50 mM.

In accordance with the invention, the fluid containing the HSP or HSP's to be recovered is preferably provided with at least one of the additives mentioned above and thereafter subjected to a precipitation step. An HSP containing fraction is thus obtained wherein some impurities are at least partially removed. The HSP fraction may be the precipitate or the supernatant..

Very suitable is differential precipitation. The term differential precipitation is used herein to describe a precipitation procedure, wherein first components other than HSP are preferentially precipitated and preferably isolated from the supernatant (comprising HSP) in at least one precipitation step and in general eventually a precipitate comprising the desired protein (HSP), is formed in a further precipitation. The differential precipitation is in practice carried out by gradually or stepwise increasing the saturation degree or concentration of a precipitation agent. The saturation degree may be increased by adding precipitation agent, by changing the temperature, by addition of another solvent or a combination thereof.

The temperature during precipitation and the rest of the purification process may be ambient (*e.g.* about 25°C or less). For practical reasons (stability of the HSP), the temperature is preferably below about 10°C, more preferably between about 0 and about 4°C.

Suitable precipitation agents include inorganic salts - in particular ammonium sulphate, sodium chloride, and other halogenides, sulfates and phosphates of alkali metals and ammonia, organic salts, such as acetates and citrates of alkali metals, and organic solvents such as acetone, acetonitrile, and alcohols (in particular methanol, ethanol, isopropanol).

The precipitation may be carried out with one precipitation agent or with several. For instance in a first step a relatively mild agent (*e.g*. NaCl) may be used at a saturation degree or concentration at which the HSP's remain substantially dissolved and thereafter a precipitation agent may be used that facilitates precipitation of substantial amounts of HSP, such as ammonium sulphate or acetone.

Very good results have been achieved with a differential precipitation wherein first an impurity is precipitated in one or more steps by using a precipitation agent, preferably ammonium sulphate, in a relatively low saturation degree (sufficiently low to maintain the majority of HSP in solution), in particular at a saturation degree in the range of about 10 to about 40 %, more in particular in the range of about 30 to about 40 % saturation, removing the precipitate from the supernatant, and then increasing the saturation degree to a relatively high degree (sufficiently high to precipitate at least a substantial amount of the HSP), in particular at a saturation degree in the range of about 45 to about 80 % more in particular in the range of about 50 to about 70 % saturation. Saturation degrees for specific compounds can routinely be determined. Further saturation points for specific compounds are known in the art.

In principle it is possible to resolve the precipitate comprising the HSP and perform subsequent precipitation procedure. However, very good results have been obtained when using only one precipitation procedure,
wherein the resultant fraction containing HSP is further processed.

The skilled person will know how to alter the regime of changing the saturation degree and/or concentration of the precipitation agent(s), depending upon the agents used based upon common general knowledge and the information disclosed herein.

The precipitation, in particular the differential precipitation, is preferably carried out at an alkaline pH, more preferably at a pH in the range of about 0.5 - 1.5 above neutral pH. Thus it has been found that release of proteases from vacuoles in the plant material is reduced and/or the protease is less active than at acidic pH.

Despite the low activity of proteases an alkaline pH it has been found advantageous that the precipitation is carried out in the presence of a protease inhibitor, even when the precipitation is carried out at an alkaline pH.

Further, the precipitation is preferably carried out in the presence of at least one additive selected from buffers (preferably buffering the solvent at an alkaline pH) protease inhibitors, protein stabilisers, antioxidants and anti-denaturants and the like, as specified above.

In order to facilitate removal of a phenol, a polyphenol and/or a lipid from the fluid, preferably use is made of a lipid and/or (poly)phenol adsorbent. In principle, such removal may take place before or after the precipitation in a separate step. However, very good results have been achieved with a method wherein such removal forms a part of the precipitation, in particular the differential precipitation. This can be achieved by adding a lipid and/or polyphenol adsorbent that precipitates at a different (in particular a lower) saturation degree of the precipation agent than HSP. A preferred example of such absorbent is poly(vinylpyrrolidone) (PVP) or betonite. PVP is preferably added to the fluid at a concentration in the range of about 0.5-10 % (w/v) more preferably between about 1.5 and about 3% (w/v). The Molecular weight of PVP is preferably in the range of 10 000 to 360 000 g/mol more preferably between 30 000 and 60 000 g/mol

The precipitates formed during the precipitation can by isolated from the supernatant in any way, *e.g.* by centrifugation.

A precipitate comprising HSP is preferably reconstituted in a solvent, *e.g.* such as an extraction solvent described above or a eluent suitable for further separation, as described herein.

Before ion-exchanging, the reconstituted HSP (or supernatant comprising HSP) is preferably subjected to a dialysis or another means (*e.g.* size exclusion chromatography) of removing precipitating agents and other relatively small components from the HSP fraction. The dialysing medium may be the same solvent as the solvent wherein the HSP precipitate was reconstituted, or another fluid but having a lower osmolarity Dialysis can be carried out in batch procedures using dialysis tubings and in co-current and counter-current flow high flux procedures. The membranes can be from a variety of materials, including cellulose acetate. In practice good results have been achieved with a molecular weight cut-off in the range of 1 000 to 70 000 g/mol, preferably between 30 000 and 50 000 g/mol

The reconstituted HSP is further subjected to an ion-exchange separation, optionally after having been separated from undissolved particles (*e.g.* by filtration and/or centrifugation).

Examples of suitable ion-exchange separations included weak ion exchange and strong ion exchange separation. The terms weak ion exchange and strong ion exchange are generally known in the art. A weak ion exchange material is a material with a comparatively low charge density at the working pH. A strong ion exchange material has in contrast usually a higher charge density and a pKs of its functional groups further away from the neutral range. More in particular, a weak-anion exchange material in practice contains positively charged functionalities above a specific pH, whereas a strong ion-exchange material in practice contains positively charged functionalities at any pH commonly employed in chromatography.

Very suitable is ion-exchange chromatography (including chromatography based techniques such as solid phase extraction), in particular anion exchange chromatography. However, in principle, any other ion exchange technique, *e.g.* electrophoresis, may be used.

Weak-ion exchange may very suitably be used for partial or complete removal of bulk proteins from crude. Weak-ion exchange is preferably carried out before the affinity separation because it allows the application of pre-purified HSP containing material to the affinity material. In particular weak-ion exchange has been found suitable to separate enzymes such as phosphatases from the HSP and thus reduce the risk of adherence of such enzymes to the affinity material, which would be highly detrimental to the performance of the affinity material.

Preferred weak-ion exchange chromatography conditions include neutral to slightly alkaline pH, low to medium concentration of inorganic salts and suitable concentrations of agents capable of reducing unspecific interactions between the protein of interest and the column material or other bulk proteins.

Examples of suitable ion exchange materials include fibrous gels, microcrystalline gels, or beaded gels. These may be made of *e.g.* any of the materials selected from agarose, sepharose, cellulose, silica, and polyacrylamide that are derivatised to carry positive groups. Preferred weak anion exchange materials, are weak anion exchange materials comprising teriairy amine groups, such as dialkylamino groups. Of these materials a material comprising diethylaminoethyl (DEAE) groups, dimethylaminomethyl (DMAE) groups or the like are particularly preferred. Very good results have been achieved with DEAE based exchange materials. More preferably, the weak ion exchange material is selected from the group consisting of DEAE cellulose, DEAE sepharose, and DEAE fractogel, because of the high flow rates and the low unspecific interactions with proteins.

The weak ion exchange separation is preferably carried out with an eluent having a neutral to slightly alkaline pH, such as in the range of 7-8.5 (as measured at 25 °C), more in particular about 7.4-8 (as measured at 25 °C) and a comparatively low total ionic strength in the range between 50 and 100 mM. The skilled person will know how to choose a suitable buffer. Preferred buffers include trishydroxymethyl aminomethane (TRIS), N-[2-Hydroxyethyl]piperazine-N'-[2-ethanesulfonic acid] (HEPES), 3-[N-morpholino]propanesulfonic acid (MOPS) and phosphate buffered saline.

The concentration of the buffer, in particular of any of said preferred buffers, is preferably in the range of about 10 to about 500 mM more preferably about 50 to about 100 mM.

The eluent preferably contains one or more additives, such as mentioned above.

Preferably at least one additive selected from protein stabilisers - in particular sucrose, glycerol, potassium, magnesium or any combination thereof - and protease inhibitors - in particular Phenylmethyl Sulfonyl Fluoride (PMSF), Pefabloc SC™, leupeptin, pepstatin, ethylenediaminetetraacetic acid (EDTA), ethylene glycol-bis(β-aminoethyl ether)-N,N,N',N'tetraaceticacid (EGTA) or any combination thereof - is present in the eluent.

Very suitable is an eluent comprising any of the following compounds, alone or in combination, in the concentration ranges indicated in the following table.

| | typical | preferred |
|---|---|---|
| protein stabiliser | 0-30% | 10-20% |
| e.g. sucrose | 0-30% | 10-20% |
| glycerol | 0-30% | 5-20% |
| Magnesium | 0.1-50 mM | 1-10 mM |
| Potassium | 0.1-50 mM | 1-10 mM |
| PMSF | 0.1-5 mM | 0.5-2mM |
| Pefabloc SC | 0.04-10 mM | 0.4-4 mM |
| leupeptin | 0.1-5 µM | 0.2-1 µM |
| pepstatin | 0.1-5 µM | 0.2-1 µM |
| EDTA | 0.1-50mM | 0.5-5mM |
| EGTA | 0.1-50mM | 0.5-5mM |

Very good results have been achieved by a gradient elution weak anion-exchange chromatography procedure, wherein gradually the ionic strength is increased, by adding a suitable displacing agent, usually anions in the form of a salt, for instance KCl. Above a certain threshold concentration the displacing agent, such as the anions of the salt displace HSP from the affinity material (resin). The amount of the displacing agent is preferably gradually increased after application of the HSP from about 0 M at the start of the separation to a value in the range of 0.05-1 M . Very good results have been achieved with an increase from about 0.005 M to a value in the range of 0.3-0.6 M .he velocity at which the gradient is applied is preferably in the range 0.1 to 0.8 cm/min.

The temperature during weak-ion separation is preferably at least about -5°C, more preferably at least about 0 °C, even more preferably at least about 2°C. For practical reasons the temperature is preferably about 10°C or less, more preferably about 4°C or less.

Optionally, the weak-ion exchange separation wherein the HSP is immobilised on the weak-ion exchange material, is preceded with another weak-ion exchange step wherein the HSP does not substantially adhere to the material, but impurities/bulk proteins with a higher affinity for the material than HSP may. This can be effected by using an eluent with a relatively high ionic strength, such as described above for the eluent that is used to mobilise the immobilised HSP from the weak ion exchange material, in particular a buffer with at least about 50 mM of displacing agent (such as chloride). At least part of the displacing agent in the eluent with the HSP is thereafter preferably removed from the eluent before passing on the weak-ion exchange step wherein the HSP is immobilised or at least retained on the column. Suitable means, *e.g.* dialysis are generally known in the art.

The ion-exchange material in this step wherein the HSP remains in the eluent phase without substantial retention may be of the same chemical structure as described above. Preferably, the material is more coarse than the step wherein the HSP is immobilised to the weak exchange material and preferably has a longer column length.

An advantage of such a preceding weak-ion exchange step is that it allows removal of a part of the proteins that will thus not interfere in the weak-ion exchange step wherein the HSP is immobilised or retained.

Strong-ion exchange is preferably carried out after the affinity separation. First, this has been found advantageous with respect to achieving a high resolution, which is found to be higher at a lower total protein concentration and when most of the bulk proteins have already been removed.

Second, HSP's tend to bind to denatured proteins during the purification. Separation thereof has been found much improved in the presence of a nucleotide such as ATP or the like. Addition of a nucleotide such as ATP before the affinity purification is thought too be risky because of the presence of a number of ATP dependent proteases that will be activated.

Third, the strong-ion exchange step has in addition been found to be useful to concentrate the HSP to a suitable concentration at which it is more stable and can be stored over a extensive time.

Preferred strong-ion exchange materials include beaded pressure resistant gels such as crosslinked agarose, sepharose and silica derivatised with functional groups such as trimethyl ammoniumethyl (TMAE), (*e.g.* MonoQ™, Resource Q™, TMAE fractogel™).

Other separation conditions for the strong-ion exchange separation (in particular the chosen eluent and other separation parameters) may be chosen such as specified for the weak-ion exchange separations. The exact conditions may be different or the same. The skilled person will know how to choose suitable conditions based upon the information disclosed herein and common general knowledge.

The affinity separation has been found very suitable to remove one or more bulk proteins such as rubisco, chlorophyll and the like.

An affinity separation is preferably carried out by affinity chromatography (including an affinity chromatography based technique such a solid phase extraction based upon affinity extraction, electrokinetic chromatography and the like).

Preferably, the affinity separation comprises affinity chromatography with a nucleotide coupled to a matrix.

Preferred matrices include agarose, sepharose, polyacrylamide, silica, and cellulose. Very good results have been achieved with agarose as a matrix material.

In principle any type of nucleotide, in particular any nucleotide triphospate (NTP) or nucleotide diphosphate (NDP), may be used for the affinity separation. Very good results have been realised with an adenosine based affinity separation, in particular an ATP or ADP based affinity separation. An adenosine affinity material, and in particular ATP affinity material has been found very suitable for recovering an HSP with a molecular weight in the range of 60-90 kD, in particular for recovering HSP70 or HSP60.

The ATP may be linked via the C8, N6 of the adenine, the 02' or O3' of the ribose or via the γ-phosphate over a spacer of 2 to 30 carbons to the matrix. Very good results for the purification of Hsp70 have been obtained with ATP linked via the C8 of the adenine over a 6 to 9 carbon spacer to a matrix, preferably an agarose matrix

The affinity material may suitably be equilibrated with a buffer as described for the (initial) buffer in the ion-exchange separation, before contacting fraction from which the HSP is to be recovered with the material.

The HSP, preferably in the same or a similar buffer as the affinity material is immobilised on the affinity material. The temperature during affinity separation is preferably at least about -5 °C, more preferably at least about 0 °C, even more preferably at least about 2 °C. For practical reasons the temperature is preferably about 10 °C or less, more preferably about 4°C or less.

The immobilisation is preferably done at a ratio of about 1-10 mg HSP to 1 ml, *e.g.* at about 5 mg/ml affinity material (preferably an ATP based material for instance ATP agarose). This ratio is based upon a loading density of 5 µmole nucleotide such as ATP per ml affinity material

After immobilisation the affinity material may be washed.

The washing solvent and the eluent for the HSP are not particular critical. In practice a buffer is present that buffers at the given pH of 7.4 to 8.0 (based upon an aqueous system). The salt concentration is preferably relatively high during one wash and relatively low during the final wash.

Additionally, the temperature is preferably about 0-4°C to avoid nucleotide(ATP) hydrolysis.

Suitable buffers include buffers as indicated above, that are suitable to be used as an (initial) eluent for the ion exchange and/or a buffer as indicated above that is suitable to be used as the (final) eluent for the ion exchange.

Preferably, the last washing step of the affinity material is with a buffer such as an initial buffer as defined for the ion exchange.

The HSP is eluted from the affinity material with an eluent comprising a displacing component, having a similar or higher affinity for the affinity material than the HSP and thereby competing with the HSP for binding to the affinity material. Alternatively, the displacing component could have a similar or higher affinity to the HSP than the affinity material thereby competing with the affinity material for binding to the HSP. Preferred examples thereof are nucleotides. In a highly preferred method the nucleotide is the same as the nucleotide on the affinity material. The concentration of the displacing component, such as the displacing nucleotide is preferably in the range of about 0.1 to about 10 mM.., more preferably in the range of about 1 to about 3 mM.. The eluent further preferably comprises a buffer and one or more other components such as defined for the initial buffer when describing the ion exchange procedure.

An affinity separation is preferably carried out between a weak ion-exchange and a strong-ion exchange separation.

After carrying out the affinity separation and ion-exchange separation(s), the purified product with the HSP may be directly used or further processed.

For instance, a further purification may be carried out, *e.g.* to remove larger or smaller proteins than the HSP of specific interest. Suitable size separation procedures are generally known in the art and include size exclusion chromatography, dialysis, and native state electrophoresis.

The invention will now further be elucidated by the following example.

### Example

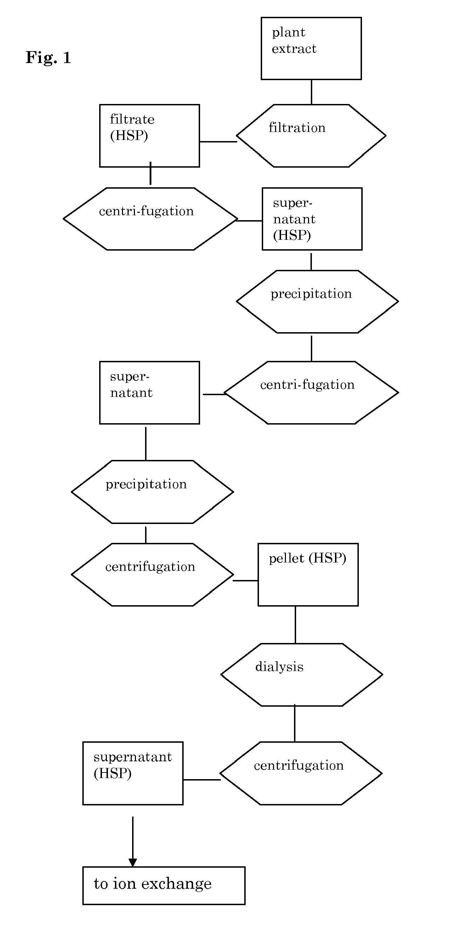

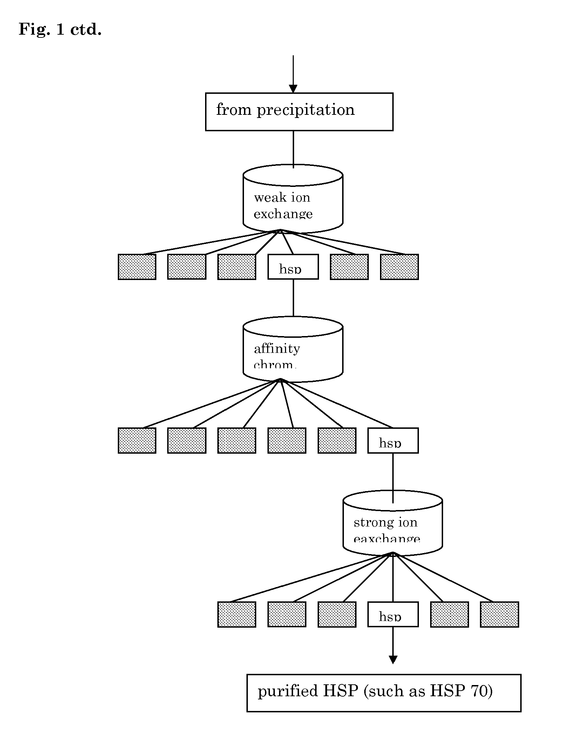

The plants (5 kg, luzerne) were disrupted in 6l 1×-extraction buffer (50 mM Tris·HCl pH 8, 50 mM (NH₄)₂SO₄, 5 mM MgCl₂, 10 mM EDTA, 10 % sucrose, 1.5 % poly(vinylpyrrolidone) K30, 1 mM phenylmethylsulfonylfluoride (PMSF), 14 mM β-mercaptoethanol, 20 mM sodium diethyldithiocarbamate) using a Warring blender, at 0-10 °C. The crude extract was filtered through cheese cloth and centrifuged. The cleared supernatant was subjected to the differential ammonium sulfate precipitation at 0-10°C. First solid powdered ammonium sulfate was added to the extract to a final saturation of 35% and precipitate and soluble proteins including HSP70 were separated by centrifugation. Subsequently, another portion of ammonium sulphate was added to the supernatant to reach a final saturation of 65% and precipitate, now containing HSP70, and soluble proteins were again separated by centrifugation. The 65 % pellet was dissolved in 150 ml buffer A (25 mM HEPES ·KOH pH 7.6, 5 mM KCI, 5 mM MgCl₂, 5 % glycerol), dialyzed twice against buffer A, and clarified from insoluble protein by centrifugation. The supernatant was applied to the equilibrated 250 ml DEAE- anion exchange column and eluted with a linear gradient from 0 to 50 % buffer B (= buffer A + 1 M KCl). The collected 10 ml fractions were analysed by SDS-polyacrylamide gel electrophoresis followed by Coomassie Brillant Blue staining and immunoblotting using HSP 70 specific antisera. The Lucerne HSP70 containing fractions were pooled and incubated with 0.5 ml in buffer A equilibrated ATP-affinity chromatography material at 0-4 °C for 30 min. The ATP affinity chromatography material was washed extensively using buffer A, buffer B, and again buffer A and lucerne HSP70 eluted as before with buffer A containing 5 mM ATP and 5 mM MgCl₂. The eluate was applied to a 1 ml TMAE strong anion exchange column and eluted using a linear gradient from 0 to 50 % buffer B. After this step the lucerne HSP70 was already found to be pure. Alternatively, HSP70 containing fractions from the ATP affinity chromatography material was applied to a superdex 200 size exclusion column equilibrated in buffer A and subsequently to a TMAE strong ion exchange column. Fig. 1 summarizes the purification scheme as a flow chart.

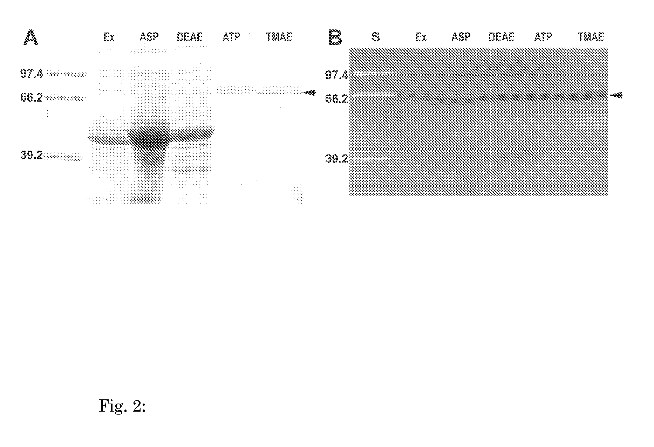

Fig. 2 shows an SDS-polyacrylamide gel with samples of the individual steps of the HSP70 purification. It is a Coomassie Brillant Blue stained SDS-polyacrylamide gel (A) and immunoblot (B) of the fractions of the individual purification steps. The arrowheads indicate the position of HSP70s. The numbers indicate the molecular weight of standard proteins

Legend: Ex= extract; ASP= pellet of precipitation; DEAE= pooled HSP70 containing fractions after DEAE chromatography; ATP = pooled HSP70 containing fractions after ATP affinity chromatography; RQ=TMAE chromatography pooled HSP70 containing fractions after strong ion exchange chromatography. Both bands visible are HSP70s since a slightly different electrophoretic mobility of the constitutive and the heat inducible form of HSP70 chaperones is frequently observed.

The functionality of the purified protein can be determined by measuring the ATPase activity including the stimulation of the ATPase activity by a DnaJ cochaperone and/or by determining the chaperone activity by measuring the refolding of a heat or chemically denatured protein.

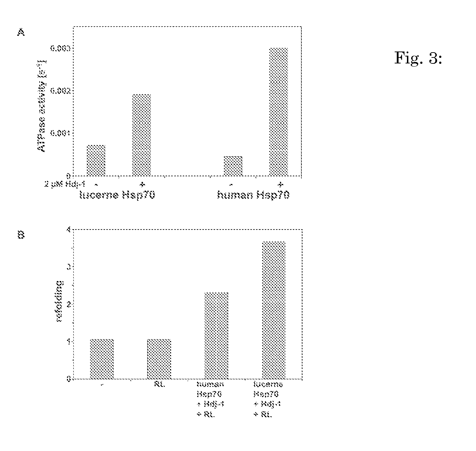

To determine the ATPase activity we incubated in a 20 µl reaction volume of buffer A 1 µM Hsp70 purified from lucerne or 1 µM human Hsp70 as a control with 250 µM, 3.7 kBq of radioactive [α-³²P]ATP in the absence or presence of 2 µM Hdj-1. The decrease of ATP and increase of ADP was followed over 3 hours by analysing aliquots by thinlayer chromatography on PEI-cellulose TLC plates (Merck) developed with a mixture of 10 % acetic acid and 400 mM LiCl. The amount of radioactive ATP and ADP was quantified using a phosphoimager (FLA3000, Fuji) and ImageReader and MacBase software (Fuji). The ratios of ATP/(ATP +ADP) were blotted against time and the resulting data fitted by linear regression analysis using the Grafit program (Erithacus). The determined value of 7.2 ·10⁻⁴ s⁻¹ for the basal ATPase rate of HSP70 from lucerne is in the normal range of other HSP70s determined so far (3 ·10⁻⁴ - 3·10⁻³ s⁻¹) (Fig. 3A). The addition of Hdj-1 to the Hsp70 purified from Lucerne preparation lead to a stimulation of the ATPase activity by a factor of 2.6 as compared to 3.0 for the human constitutive HSC70.

We tested the chaperone activity of the lucerne HSP70 preparation in the following way. By incubation at 42°C for 40 min we heat denatured firefly luciferase (80 nM) in refolding buffer (25 mM Hepes ·KOH pH 7.6, 100 mM KCl, 10 mM MgCl₂, 5 mM DTT, 2 mM ATP) containing either no additions, or 1 µM human Hsp70 + 0.2 µM Hdj-1, or 1 µM lucerne HSP70 + 0.2 µM Hdj-1. After temperature downshift 5 % rabbit reticulocyte lysate according to the well established standard conditions was added where indicated, and the luciferase activity was followed over a time span of 90 min by transferring 2 µl aliquots of the refolding reaction into 125 µl assay buffer (25 mM Glycylglycin, pH 7,4, 5 mM ATP, 100 mM KCl, 15 mM MgCl₂) containing 80 µM luciferin as substrate. Active luciferase oxidates its substrate luciferin whereby light pulses are emitted. These light pulses were measured using a lumat (Berthold) and related to the amount of active luciferase. In the absence of HSP70 the measured luciferase activity did not surpass on tenth of the original activity. In the presence of Hdj-1, rabbit reticulocyte lysate, and our lucerne HSP70 preparation 3.7-fold more denatured luciferase was refolded into the native state within 60 min (Fig. 3B).

Spontaneous refolding and refolding in the presence of rabbit reticulocyte lysate was also assessed and found to be 10 %.

This experiment clearly demonstrates that the lucerne HSP70 has chaperone activity and cooperates with the human cochaperone Hdj-1 and components of the reticulocyte lysate very similar to human HSP70. As such it is capable of enhancing the refolding of proteins.

## Claims

1. Plant heat shock protein showing chaperone activity in combination with a vertebrate cochaperone for use as a medicament.

2. Plant heat shock protein for use according to claim 1, wherein the medicament is for the treatment of an indication selected from inflammatory diseases, autoimmune diseases, neurodegenerative diseases, cancer, necrotic processes, regenerative processes after injuries, lesions and hypoxic stress conditions.

3. Plant heat shock protein for use according to claim 1 or 2, wherein the plant heat shock protein shows chaperone activity in combination with a cochaperone of a mammal.

4. Plant heat shock protein for use according to claim 3, wherein the plant heat shock protein shows chaperone activity in combination with a human cochaperone or a functional analogue thereof.

5. Plant heat shock protein for use according to any of the previous claims, wherein the plant heat shock protein is selected from HSP60, HSP70 and HSP90.

6. Plant heat shock protein for use according claim 5, wherein the plant heat shock protein is HSP70.

7. Plant heat shock protein for use according to any of the previous claims, wherein the plant heat shock protein originates from a lucerne plant.

8. Plant heat shock protein for use according to any of the previous claims, wherein the cochaperone activity of the heat shock protein is such that *in vitro* refolding of a denatured firefly luciferase is enhanced in comparison to spontaneous refolding, and the refolding is preferably at least doubled in the presence of HSP and cochaperone, as determined 90 min. after shift to refolding conditions at 30°C after optional removal of denaturants.

9. Plant heat shock protein for use according to any of the previous claims, wherein the medicament further comprises a cochaperone.

10. Plant heat shock protein for use according to claim 9, wherein the cochaperone is a DnaJ cochaperone or a plant homologue thereof, preferably a cochaperone selected from the group of Hdj1 and Hdj2.

11. Plant heat shock protein for use according to claim 9 or 10, wherein the molar ratio of cochaperone to plant heat shock protein is 0.1 - 1.

12. Plant heat shock protein for use according to any of the previous claims, wherein the medicament is for *in vivo* protein repair.

13. Complex of a vertebrate cochaperone and a plant heat shock protein showing chaperone activity in combination with a vertebrate cochaperone.

14. Composition comprising a vertebrate cochaperone and a plant heat shock protein showing chaperone activity in combination with the vertebrate cochaperone.

15. Composition according to claim 14, wherein the composition comprises a cochaperone of the DnaJ family, preferably Hdj-1 or Hdj-2.
